# FASCICULE DE BREVET EUROPEEN

(11) **EP 0 703 922 B1**
(45) Date de publication et mention de la délivrance du brevet: **17.05.2000**
(21) Numéro de dépôt: 94918916.1
(22) Date de dépôt: 13.06.1994
(51) Int. Cl.: C07K 1/00, A61K 39/395

(54) **PROCEDE DE PRODUCTION DE CONCENTRE D'IMMUNOGLOBULINES G**
VERFAHREN ZUR HERSTELLUNG VON IMMUNOGLOBULIN G KONZENTRAT
METHOD FOR PRODUCING IMMUNOGLOBULIN G CONCENTRATE

(30) Priorité: 14.06.1993 FR 9307128
(43) Date de publication de la demande: 03.04.1996
(73) Titulaire: ASSOCIATION POUR L'ESSOR DE LA TRANSFUSION SANGUINE DANS LA REGION DU NORD, 59000 Lille (FR)
(72) Inventeur: BURNOUF, Miryana, F-59136 Wavrin (FR); DERNIS, Dominique, F-59520 Marquette-lez-Lille (FR); BONNEEL, Patrick, F-59000 Lille (FR); BURNOUF, Thierry, F-59136 Wavrin (FR)
(74) Mandataire: Lhuillier, René
(86) Numéro de dépôt international: FR9400699
(87) Numéro de publication internationale: WO9429334

(56) Documents cités:
- EP-A- 0 121 468
- EP-A- 0 268 973
- EP-A- 0 270 025
- EP-A- 0 440 483
- WO-A-89/05157
- DE-A- 2 641 840
- DE-A- 3 208 523
- DE-C- 4 118 912
- US-A- 4 136 094
- US-A- 4 806 346
- US-A- 4 841 024

## Description

La présente invention concerne un concentré d'immunoglobulines G à usage thérapeutique et le procédé de production dudit concentré.

Diverses préparations d'immunoglobulines polyvalentes sont déjà utilisées depuis longtemps. D'une manière générale, elles sont préparées à partir d'un "pool" de sérums de 2.000 à 5.000 donneurs ce qui assure la présence de tous les anticorps normalement présents dans l'ensemble de la population d'une contrée choisie.

Ces préparations d'immunoglobulines sont préparées selon la méthode classique de Cohn plus ou moins modifiée, c'est-à-dire par précipitation à l'éthanol. Ce procédé présente un désavantage important dû au traitement à l'éthanol qui provoque une certaine dénaturation des proteines et la formation d'agrégats de polymères d'immunoglobulines. Ceux-ci présentent un risque thérapeutique en provoquant l'activation du système complément et des réactions anaphylactiques. Ces préparations ne peuvent donc pas être utilisées en injections intraveineuses mais seulement en intramusculaires, ce qui en limite la dose injectable et l'efficacité.

Différents traitements sont déjà appliqués pour contourner ce problème : le clivage des immunoglobulines par la pepsine ou par la plasmine, un traitement à la β-propiolactone ou avec des agents réducteurs et alkylants, ou à pH 4, ou avec du PEG pour précipiter les agrégats.

La Demanderesse a préféré éviter ces traitements enzymatiques ou chimiques et a supprimé l'étape de précipitation à l'éthanol. Elle a ainsi mis au point un procédé qui comprend une succession de séparations chromatographiques et qui ne comporte aucune précipitation.

La préparation de concentrés d'immunoglobulines par chromatographie a déjà été expérimentée par Friesen et al. (Friesen - Joint IABS/CSL Symp. on Standardization in Blood Fractionation - Australia 1986 - Develop. biol. Standard.67,1987, 3-13 ; Immunoglobulins - Pub. Central Lab.Netherlands Red Cross 1988 - Ed. Krynen, Strengers, Van Aken) qui utilisent une ou deux chromatographies d'échanges d'anions :
- Une chromatographie unique sur DEAE-Sephadex permet la préparation de γ.globulines spécifiques à partir de serums hyperimmuns, mais elle n'est applicable qu'à de petits volumes.
- Une chromatographie sur DEAE-Sepharose suivie d'une chromatographie sur DEAE-Sephadex permet de préparer de plus grands volumes et est applicable à la préparation d'immunoglobulines polyvalentes, mais le procédé ne s'est pas révélé rentable à l'échelle industrielle.

Un procédé de purification des IgG par chromatographie est décrit dans EP-0 268 972, à partir d'une fraction de plasma prépurifiée de préférence par précipitation à l'éthanol(à l'exception d'un des exemples).

Le procédé comprend ensuite une diafiltration ou une chromatographie de tamisage moléculaire sur gel (de type Sephadex...), puis une chromatographie d'échange d'anions, puis une séparation ou inactivation des enzymes par chromatographie d'affinité ou par addition d'antithrombine III. Le procédé comprend ensuite une stérilisation à la β-propiolactone et aux UV.

La purification des immunoglobulines pose des problèmes particuliers, outre ceux qui ont déjà été cités plus haut, parce que leur activité biologique est liée à leur intégrité structurale, or cette activité ne se mesure pas simplement, comme une activité enzymatique. Ainsi, par exemple, pour un anticorps spécifique, à taux égal en anticorps mesuré par ELISA, le pouvoir de neutralisation de l'infectivité d'un virus varie fortement d'un concentré à l'autre, en fonction du procédé de préparation.

La Demanderesse s'est donc attachée a mettre au point un procédé de purification des immunoglobulines non dénaturant, applicable à l'échelle industrielle (par exemple sur des lots de serum de plus de 500 litres) et qui permet, en outre, la récupération des autres protéines plasmatiques d'intérêt thérapeutique.

Le procédé de production d'un concentré d'immunoglobulines, selon la présente invention, ne comprend pas de précipitation à l'éthanol et comprend une succession d'étapes chromatographiques au cours desquelles les immunoglobulines restent toujours en phase liquide et à un pH compris entre 5,5 et 7,8. Le procédé comprend également une étape d'inactivation virale, par exemple par traitement au solvant-détergent.

Le procédé selon la présente invention s'applique au plasma total ou, de préférence, au surnageant du cryoprécipité.

Il s'applique aussi bien à de grands volumes de "pool" de plasmas destinés à la préparation d' immunoglobulines polyvalentes qu'à de plus petits volumes de plasmas hyperimmuns destinés à la préparation d' immunoglobulines spécifiques.

Le plasma ou la fraction de plasma de départ est de préférence soumis à une étape de prépurification, avant la mise en oeuvre du procédé proprement dit ; celle-ci est effectuée par filtration sur une série de cartouches filtrantes de porosité de 0,5 à 0,2µ, constituées de cellulose et de perlites, portant des charges négatives, et d'une faible quantité de résine chargée positivement (filtres Zeta plus® fournis par Cuno - USA) . Ces filtres, grâce à leur charge négative, permettent l'adsorption et donc ici l'élimination du Facteur XI qui, sans cette étape de prépurification se retrouve copurifié avec les immunoglobulines ce qui peut entrainer une intolérance au produit final et des phénomènes d'hypotension.

Le procédé comprend éventuellement une ou plusieurs étapes de prépurification qui permettent d'éliminer certaines protéines plasmatiques et ainsi de diminuer la taille des colonnes de chromatographie ultérieures, ce qui est particulièrement avantageux pour les grands volumes. Ainsi la fraction de protéines peut être prépurifiée en "batch" en présence d'un gel échangeur d'anions, de type DEAE-Sephadex®, qui permet la rétention et donc l'élimination du Facteur IX, du Facteur VII, de la protéine C. Elle peut ensuite être injectée sur une colonne de gel échangeur d'anions, de type DEAE-Sepharose®, qui permet la rétention et l'élimination de l'albumine et de l'α.antitrypsine. Ces dernières peuvent être désorbées et concentrées selon des méthodes connues, et avec un rendement particulièrement élevé grâce au procédé tel que décrit pour la présente invention.

La fraction protéique constituant le filtrat de la colonne peut éventuellement être soumise à une chromatographie sur une colonne d'héparine-Sepharose® avant d'être soumise à la chromatographie suivante, afin d'éliminer l'antithrombine III.

Le procédé selon la présente invention comprend une étape de dessalage, soit par ultrafiltration, soit par chromatographie de tamisage moléculaire, dans laquelle le filtrat des étapes de prépurification éventuelles est injecté sur une colonne de gel de type dextran réticulé, par exemple du Sephadex®-G25, équilibré en tampon Tris-HCl 0,022M à pH 7,8 et la fraction contenant les protéines plasmatiques est récupérée.

Ladite fraction protéique est ensuite injectée sur une colonne de chromatographie constituée d'un gel échangeur d'anions, plus particulièrement d'un gel greffé de groupements DEAE. On obtient un rendement particulièrement efficace avec un gel de type acrylamide réticulé comme le DEAE-Trisacryl® Plus LS (Sepracor-IBF, réf. 262080), équilibré en tampon Tris-HCl 0,025M à pH 7,8. Cette colonne adsorbe pratiquement toutes les protéines plasmatiques sauf les immunoglobulines G. Ces dernières se retrouvent dans le filtrat et peuvent être concentrées à environ 50 g/l.

La solution concentrée d'immunoglobulines est ensuite soumise à un traitement d'inactivation virale, par exemple, par un traitement en présence de solvant-détergent et de préférence de TnBP (tri(n-butyl)phosphate) 0,3 % et de Tween®80 (polyoxyethylenesorbitane-monooléate) 1 %, à 25°C sous légère agitation pendant au moins 6 heures.

Le procédé selon la présente invention comprend ensuite une chromatographie sur gel échangeur de cations, plus particulièrement sur un gel greffé de groupements carboxyméthyle (CM) . On obtient un rendement parciculièrment efficace avec un gel de type CM-Trisacryl® LS (Sepracor, réf. 260280) équilibré en tampon acétate de sodium 0,024M à pH 5,5. l'élimination du solvant-détergent dans le filtrat. Les immunoglobulines sont ensuite désorbées et éluées par augmentation de la force ionique du tampon à 0,15 - 0,19 M NaCl.

Elles sont additionnées de saccharose à 110g/l, conditionnées et lyophilisées.

L'analyse du concentré d'immunoglobulines G obtenu par le procédé décrit plus haut confirme qu'il est dépourvu d'agrégats d'immunoglobulines G, d'immunoglobulines A et, de manière surprenante, qu'il est également dépourvu d'immunoglobulines E ou qu'il n'en contient plus qu'un taux inférieur à 10 % du taux présent dans le plasma, ce qui lui confère un avantage thérapeutique supplémentaire.

La présente invention concerne donc un concentré d'immunoglobuline G d'origine plasmatique dépourvu d'agrégats d'immunoglobulines G et d'immunoglobulines E, ainsi que d'IgA.

Ces propriétés le rendent donc particulièrement adapté à une injection par voie intraveineuse et il est formulé de manière appropriée en vue de ladite injection.

Les exemples suivants décrivent des modes de mise en oeuvre du procédé.

### Exemple 1

Le matériau de départ est un pool de 500 litres de plasma de donneurs sains, choisis au hasard dans une population normale.

Après cryoprécipitation selon les méthodes classiques, le surnageant est récupéré pour être soumis aux différentes étapes de chromatographie

### 1.A Chromatographie de dessalage.

Cette chromatographie est effectuée sur une colonne GF 04-06 (Sepracor®) de 65 litres de Sephadex®G25 équilibrée en tampon Tris HCl 0,022M à pH 7,8, à un débit de 450 litres/heure.

Le filtrat est suivi par densitométrie et la fraction contenant l'ensemble des protéines est récupérée.

La colonne est régénérée par lavage avec une

La colonne est régénérée par lavage avec une solution de NaCl 1M.

### 1.B Chromatographie sur gel échangeur d'anions.

Cette chromatographie est effectuée sur des colonnes GF 08015 de 65 litres de DEAE-Trisacryl® équilibrées en tampon Tris-HCl 0,025M à pH 7,8, à un débit de 150 litres/heure.

Dans ces conditions d'utilisation, les immunoglobulines G sont pratiquement les seules protéines qui ne s'adsorbent pas sur la colonne.

Le filtrat est suivi par densitométrie et la fraction protéique est récupérée. Elle est donc constituée à prés de 100 % d'immunoglobulines G.

L'albumine qui est restée adsorbée sur la colonne est éluée par addition de NaCl 0,4M dans le tampon et elle est concentrée selon des méthodes classiques.

La colonne est régénérée par lavage avec une solution de NaCl 2M.

### 1.C Traitement d'inactivation virale.

Le filtrat de la colonne précédente est concentré à environ 50g/l et soumis à un traitement classique par addition d'un mélange de solvant-détergent, pendant 6 heures à 25°C sous agitation lente.
Le mélange comprend du TnBP 0,3 % et du Tween 80 1 %.

### 1.D. Chromatographie sur gel échangeur de cations.

Cette chromatographie est effectuée sur une colonne GF 08015 de 65 litres de CM-Trisacryl® équilibrée en tampon acétate de sodium 0,024M à pH 5,5, à un débit de 20 à 100 litres/heure.

Dans ces conditions d'utilisation les immunoglobulines s'adsorbent sur le gel et la combinaison solvant-détergent et les éventuels contaminants dénaturés ou autres résidus sont éliminés dans le filtrat.

Les immunoglobulines sont ensuite désorbées par augmentation de la force ionique du tampon par addition de chlorure de sodium 0,15 à 019M

La fraction d'immunoglobulines récupérée a une concentration de 30 à 50g/l et peut donc être conditionnée telle quelle, après addition de saccharose à 110g/l.

La colonne est régénérée par lavage avec une solution de NaCl 1M.

### Exemple 2

Au procédé décrit dans l'exemple 1 on ajoute une étape préliminaire de prépurification, avant la première chromatographie.

Le surnageant du cryoprécipité est soumis à une filtration sur une batterie de 3 cartouches de filtres de porosité comprise entre 0,5 et 0,2 µ et essentiellement chargés négativement (Filtres Zeta plus ®, fournis par Cuno, Process Filtration Products, filiales de Commercial Intertech Corp. USA, et décrit dans les brevets US 4,783,262 et 4,859,340, dénommés "filtres Cuno") . Ces filtres sont constitués de cellulose purifiée, de perlites et d'une faible quantité de résine chargée positivement. D'autres systèmes de filtres disponibles dans le commerce peuvent également être utilisés.

Les filtres sont rincés en tampon citrate/phosphate comprenant du citrate de sodium, du phosphate disodique, du phosphate de potassium, du chlorure de sodium et de l'EDTA-disodique et ajusté à un pH compris entre 5,5 et 6,5 et, préférentiellement, à pH 6.

Cette étape de filtration permet la rétention et donc l'élimination du Facteur XI.

### Exemple 3

Au procédé décrit dans l'exemple 1 ou dans l'exemple 2 on ajoute une étape préalable d'adsorption sur gel échangeur d'anions, avant la première chromatographie de dessalage sur Sephadex G25.

Cette adsorption préalable est effectuée en "batch" en présence de DEAE-Sephadex® à une concentration de 1,5g/litre de plasma, durant 2 à 5 heures. Ceci permet la rétention et donc l'élimination du Facteur IX, du Facteur VII et de la protéine C. Les immunoglobulines se retrouvent dans le surnageant.

Celui-ci peut encore être soumis à une prépurification supplémentaire par chromatographie sur colonne de DEAE-Sepharose® CL6B fast flow (Pharmacia) . Le matériel plasmatique est dialysé et ajusté à une conductivité de 1,6 mS avec de l'acétate de sodium 0,025M. Le pH est ajusté à 7,6.

La colonne est équilibrée en tampon acétate de sodium 0,025M à pH 7,6.

Les immunoglobulines se retrouvent dans le filtrat.

Ce procédé a le double avantage :
- de laisser filtrer des immunoglobulines déjà prépurifiées et ainsi de permettre une réduction de la taille des colonnes chromatographiques suivantes.
- et de retenir l'albumine et l'α antitrypsine qui peuvent ensuite être purifiées, selon des méthodes connues avec un rendement particulièrement élevé.

Le filtrat contenant les immunoglobulines G prépurifiées peut encore être soumis à une chromatographie sur colonne d'héparine-Sepharose® équilibrée en tampon phosphate 0,02 M -chlorure de sodium 0,154M à pH 6,8.

Cette chromatographie permet d'adsorber et donc d'éliminer l'antithrombine III alors que les immunoglobulines G se retrouvent dans le filtrat.

### Exemple 4

On a comparé les titres en anticorps mesurés par ELISA et le pouvoir neutralisant de l'infectivité d'un virus choisi comme modèle, le cytomegalovirus, avec les immunoglobulines préparées selon les méthodes classiques de fractionnement par l'éthanol suivi d'un traitement à pH4 en présence de pepsine et selon le procédé de la présente invention.

| Mode de préparation des IgG | Titre ELISA (U/ml) | Pouvoir neutralisant (titre du CMV) |
|---|---|---|
| • Méthode traditionnelle | | |
| lot 1 | 100 | 1280 |
| lot 2 | 110 | 640 |
| lot 3 | 110 | 640 |

| • Selon la présente invention | | |
|---|---|---|
| lot 1 | 90 | 2560 |
| lot 2 | 120 | 5120 |
| lot 3 | 100 | 5120 |

Ces résultats indiquent clairement que, pour une quantité équivalente d'anticorps (en ELISA) la qualité de ceux-ci, évaluée par leur activité biologique (pouvoir neutralisant) est nettement supérieure pour les anticorps produits par voie chromatographique.

### Exemple 5

On a mesuré la quantité d'IgE présente dans le plasma de départ, dans des lots de concentré d'IgG préparés selon la méthode classique (comme rappelé dans l'exemple 4) et par la méthode chromarographique selon la présente invention.

Le tableau suivant montre qu'il ne subsiste presque pas d'IgE dans la préparation par chromatographie alors qu'elles sont plus concentrées que dans le plasma de départ dans les préparations classiques.

| | N°. de lot | IgE (UI/ml) |
|---|---|---|
| PLASMA DEPART | 99030120 | 96 |
| | 30162 | 108 |
| | 30170 | 118 |
| IgG "classiques" | 50020200 | 293 |
| | 20231 | 259 |
| | 20291 | 272 |
| | 23300 | 220 |
| | 30070 | 230 |
| | 30071 | 210 |
| | 30072 | 246 |
| | 30080 | 242 |
| | 30081 | 258 |
| | 30082 | 248 |
| | 30090 | 214 |
| | 30091 | 212 |
| | 30092 | 170 |
| | 30100 | 160 |
| | 30101 | 117 |
| | 30102 | 144 |
| | 30110 | 250 |
| | 30112 | 224 |
| | 30120 | 195 |
| | 30121 | 161 |
| | 30122 | 220 |
| | 30130 | 238 |
| | 30131 | 248 |
| | 30132 | 227 |
| IgG préparées par chromatographie | 50230050 | 12 |
| | 070 | 6 |
| | 080 | 8,1 |
| | 090 | 4,1 |
| | 100 | 7,5 |
| | 110 | 3,5 |

## Revendications

1. Procédé de production d'un concentré d'immunoglobulines G à usage thérapeutique à partir de plasma humain ou de surnageant de plasma cryoprécipité, caractérisé en ce qu'il ne comprend pas de précipitation à l'éthanol et qu'il comprend une succession d'étapes de chromatographie au cours desquelles les immunoglobulines restent toujours en phase liquide et à un pH compris entre 5,5 et 7,8, ainsi qu'une étape d'inactivation virale, l'ensemble du procédé comprenant successivement :
a- une étape de dessalage,
b- une chromatographie sur gel échangeur d'anions,
c- un traitement d'inactivation virale,
d- et une chromatographie sur gel échangeur de cations.

2. Procédé selon la revendication 1, caractérisé en ce que le matériau de départ est la fraction du plasma surnageant après cryoprécipitation.

3. Procédé selon la revendication 2, caractérisé en ce que le surnageant de la cryoprécipitation est soumis à une étape de prépurification par filtration sur une série de cartouches de filtres de porosité de 0,5 à 0,2 µ, constitués de cellulose et de perlites, portant des charges négatives, et d'une faible quantité de résine chargée positivement.

4. Procédé selon l'une quelconque des revendications 1 à 3, caractérisé en ce qu'il comprend une ou plusieurs étapes de prépurification par adsorption de certaines protéines plasmatiques sur des gels choisis parmi les gels échangeurs d'anions et l'héparine-sepharose.

5. Procédé selon la revendication 1, caractérisé en ce que l'étape a) est réalisée sur un gel de type dextran réticulé, équilibré en tampon Tris-HCl 0,022M à pH 7,8 et que la fraction comprenant les protéines plasmatiques est récupérée pour être soumise à l'étape b).

6. Procédé selon la revendication 1, caractérisé en ce que l'étape b) est réalisée sur un gel de type acrylamide réticulé, greffé avec des groupements DEAE, équilibré en tampon Tris-HCl 0,025M à pH 7,8 et que le filtrat est récupéré pour être soumis à l'étape c).

7. Procédé selon la revendication 1, caractérisé en ce que l'étape c) est réalisée par traitement au solvant-détergent pendant au moins 6 heures à 25°C.

8. Procédé selon la revendication 1, caractérisé en ce que l'étape d) est réalisée sur un gel de type acrylamide réticulé, greffé avec des groupements carboxyméthyle, équilibré en tampon acétate de sodium 0,024M à pH 5,5, et que les immunoglobulines adsorbées sur le gel sont éluées par augmentation de la force ionique du tampon à 0,15 - 0,19M NaCl.

9. Procédé selon la revendication 1, caractérisé en ce que les immunoglobulines éluées de l'étape d) sont additionnées de saccharose à 110 g/l puis lyophilisées.

## Claims

1. A process for producing an immunoglobulin G concentrate for therapeutic use, starting from human plasma or from plasma cryosupernatant,characterized in that it does not include any ethanol precipitation and that it includes a series of chromatographic steps during which the immunoglobulins always remain in a liquid phase and at a pH of between 5.5 and 7.8, and a viral inactivation step, the whole process including successively :
a - a dessalting step,
b - a chromatography on anion exchange gel,
c - a viral inactivation treatment,
d - and a chromatography on cation exchange gel.

2. A process according to claim 1, characterized in that the starting material is the supernatant plasma fraction after cryoprecipitation.

3. A process according to claim 2, characterized in that the cryoprecipitation supernatant is subjected to a prepurification step by filtration through a series of cartridges of filters with a porosity of 0.5 to 0.2 µ, made of cellulose and perlites, negatively charged, and of a small amount of positively charged resin.

4. A process according to any of claims 1 to 3, characterized in that it includes one or several prepurification steps by adsorption of some plasma proteins on gels chosen among anion exchange gels and heparin-SEPHAROSE.

5. A process according to claim 1, characterized in that step a) is performed on a cross linked dextran-type gel, equilibrated with 0.022 M TRIS-HCl buffer at pH 7.8, and that the fraction containing the plasma proteins is collected to be subjected to step b).

6. A process according to claim 1, characterized in that step b) is performed on a cross linked acrylamide type gel, grafted with DEAE residues, equilibrated with 0,025 M TRIS-HCl buffer at pH 7.8, and that the filtrate is collected to be subjected to step c).

7. A process according to claim 1, characterized in that step c) is performed by solvent-detergent treatment for at least 6 hours at 25°C.

8. A process according to claim 1, characterized in that step d) is performed on a croos-linked acrylamide-type gel, grafted with carboxymethyl residues, equilibrated with 0,024 M sodium acetate buffer at pH 5.5, and that the immunoglobulins adsorbed on the gel are eluted by increasing the ionic strength of the buffer to 0.15-0.19 M NaCl.

9. A process according to claim 1, characterized in that the immunoglobulins eluted from step d) are supplemented with 110 g/l saccharose and then freeze-dried.

## Patentansprüche

1. Verfahren zum Herstellen eines Konzentrats von Immunglobulinen G zur therapeutischen Verwendung ausgehend von menschlichem Plasma oder dem Überstand von Plasma Kryopräzipitation, **dadurch gekennzeichnet,** daß es keine Fällung mit Ethanol umfaßt und daß es eine Abfolge von Chromatographie-Schritten, in deren Verlauf die Immunglobuline stets in flüssiger Phase und bei einem pH zwischen 5,5 und 7,8 bleiben, sowie einen Virusinaktivierungsschrift umfaßt, wobei das ganze Verfahren nacheinander umfaßt:
a) einen Entsalzungsschritt
b) eine Chromatographie an einem Anionenaustauschergel
c) eine Virusinaktivierungsbehandlung
d) und eine Chromatographie an einem Kationenaustauschergel.

2. Verfahren nach Anspruch 1, **dadurch gekennzeichnet,** daß das Ausgangsmaterial die nach Kryopräzipitation überstehende Plasma-Fraktion ist.

3. Verfahren nach Anspruch 2, **dadurch gekennzeichnet,** daß der Überstand von der Kryopräzipitation einem Vorreinigungsschritt durch Filtration über eine Reihe von Filterpatronen mit einer Porosität von 0,5 bis 0,2 µ unterzogen wird, die aus Cellulose und aus Perliten, welche negative Ladungen tragen, und aus einer geringen Menge an positiv geladenem Harz bestehen.

4. Verfahren nach einem der Ansprüche 1-3, **dadurch gekennzeichnet,** daß es einen oder mehrere Vorreinigungsschritte durch Adsorption von bestimmten Plasmaproteinen an Gelen umfaßt, die ausgewählt sind aus den Anionenaustauschergelen und Heparin-Sepharose.

5. Verfahren nach Anspruch 1, **dadurch gekennzeichnet,** daß der Schritt an einem Gel aus vernetztem Dextran, das in einem 0,022M Tris-HCl-Puffer bei pH 7,8 äquilibriert ist, durchgeführt wird und daß die Fraktion, welche die Plasmaproteine umfaßt, zurückgewonnen wird, um dem Schritt b) unterzogen zu werden.

6. Verfahren nach Anspruch 1, **dadurch gekennzeichnet,** daß der Schrift b) an einem Gel aus vernetztem Acrylamid, das mit DEAE-Gruppen gepfropft ist und in einem 0,025M Tris-HCl-Puffer bei pH 7,8 äquilibriert ist, durchgeführt wird und daß das Filtrat zurückgewonnen wird, um dem Schritt c) unterzogen zu werden.

7. Verfahren nach Anspruch 1, **dadurch gekennzeichnet,** daß der Schrift c) durch Behandlung mit einem Lösungsmittel-Detergens während mindestens 6 Stunden bei 25°C ausgeführt wird.

8. Verfahren nach Anspruch 1, **dadurch gekennzeichnet,** daß der Schritt d) an einem Gel aus vernetztem Acrylamid, das mit Carboxymethylgruppen gepfropft ist und in einem 0,024M Natriumacetat-Puffer bei pH 5,5 äquilibriert ist, durchgeführt wird und daß die an dem Gel adsorbierten Immunglobuline durch Erhöhen der Ionenstärke des Puffers auf 0,15 - 0,19M NaCl eluiert werden.

9. Verfahren nach Anspruch 1, **dadurch gekennzeichnet,** daß die eluierten Immunglobuline von Schritt d) mit Saccharose bis 110 g/l versetzt und anschließend lyophilisiert werden.
